(19) Europäisches Patentamt
European Patent Office
Office européen des brevets

(11) Numéro de publication: **0 258 120 B1**

(12) **FASCICULE DE BREVET EUROPEEN**

(45) Date de publication de fascicule du brevet: **15.12.93** (51) Int. Cl.[5]: **C08F 2/32**

(21) Numéro de dépôt: **87401825.2**

(22) Date de dépôt: **06.08.87**

# (54) Polymère en poudre, son procédé de préparation et son application à l'absorption de fluides aqueux.

(30) Priorité: **14.08.86 FR 8611742**

(43) Date de publication de la demande:
**02.03.88 Bulletin 88/09**

(45) Mention de la délivrance du brevet:
**15.12.93 Bulletin 93/50**

(84) Etats contractants désignés:
**AT BE CH DE ES FR GB GR IT LI LU NL SE**

(56) Documents cités:
**DE-B- 1 123 110**
**GB-A- 2 088 392**
**US-A- 2 982 749**
**US-A- 4 070 321**

(73) Titulaire: **ELF ATOCHEM S.A.**
**4 & 8, Cours Michelet**
**La Défense 10**
**F-92800 Puteaux(FR)**

(72) Inventeur: **Mallo, Paul**
**290 J Avenue Napoléon Bonaparte**
**F-92500 Rueil Malmaison(FR)**
Inventeur: **Cretenot, Claude Lise**
**1 Rue de la Pinéde**
**F-60550 Verneuil en Halatte(FR)**

(74) Mandataire: **Chaillot, Geneviève**
**Cabinet CHAILLOT**
**21, avenue Louise de Bettignies**
**F-92700 Colombes (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 258 120 B1

## Description

L'industrie des produits à base de papier pour l'hygiène et la santé, tels que les serviettes hygiéniques et les couches pour bébés, est à la recherche de substances capables de remédier au défaut principal du papier, à savoir sa capacité insuffisante à absorber et retenir l'eau. De telles substances devraient, de manière idéale, présenter les propriétés suivantes : une capacité d'absorption et rétention d'eau, en particulier d'eau saline, élevée ; une vitesse d'absorption élevée ; constituer, après absorption d'eau, un hydrogel présentant un module élastique ("force de gel") élevé ; une capacité d'absorption d'eau, en particulier d'eau saline, demeurant élevée sous l'effet de la pression; une proportion de substances extractibles (polymères solubles et monomères résiduels) par l'eau saline aussi faible que possible; enfin il est nécessaire que de telles substances puissent être fabriquées à bas prix.

Plusieurs solutions ont déjà été proposées pour résoudre les différents problèmes posés par l'industrie des produits à base de papier pour l'hygiène et la santé. Ainsi le brevet FR-A-2.367.083 décrit un procédé de préparation d'un homopolymère d'acrylate d'un métal alcalin, spontanément réticulé, en poudre, ayant une capacité d'absorption d'eau non saline d'au moins 400 g par g de polymère sec, caractérisé en ce qu'il consiste à disperser et à mettre en suspension une solution aqueuse d'un acrylate d'un métal alcalin ayant une concentration d'au moins 40 % en poids et contenant un initiateur de polymérisation radicalaire hydrosoluble, dans un solvant hydrocarboné aliphatique liquide, en présence d'un ester d'acide gras de sorbitan ayant une valeur HLB de 3 à 6, et à polymériser l'acrylate de métal alcalin en l'absence d'un agent de réticulation. Le rapport en volume de la phase huile à la phase aqueuse varie de préférence entre 1 et 4. Selon ce procédé ledit ester d'acide gras de sorbitan est utilisé de préférence à raison de 1 à 10% en poids par rapport à l'acrylate de métal alcalin. Le polymère est alors obtenu sous forme d'une poudre ayant une dimension de particules de 10 à 120 $\mu$m. De son côté le brevet EP-B-0.036.463 décrit un procédé de polymérisation en suspension inverse d'une solution aqueuse d'acide acrylique et d'un acrylate, dans lequel le rapport molaire de l'acide acrylique à l'acrylate est de 50/50 à 2/98 et la concentration des monomères est d'au moins 40 % en poids, en présence d'un initiateur de polymérisation radicalaire soluble dans l'eau, caractérisé en ce qu'on utilise une suspension d'une solution aqueuse d'acide acrylique et d'un acrylate de métal alcalin ou d'un acrylate d'ammonium dans un hydrocarbure aliphatique ou cycloaliphatique contenant un agent tensio-actif ayant une valeur de HLB de 8 à 12. Ce document décrit un rapport en poids de l'hydrocarbure à la solution aqueuse égal à environ 1,9 et une proportion d'agent tensio-actif de préférence comprise entre 1 et 15% en poids par rapport aux monomères. Le brevet US-A-4.446.261 décrit un procédé de préparation d'un polymère réticulé, insoluble dans l'eau, sous forme de particules ayant une taille de 100 à 1000 $\mu$m et ayant une capacité d'absorption d'eau saline physiologique de 30 à 150 g/g, comprenant les étapes suivantes :

- dispersion de gouttelettes d'une solution aqueuse (1) constituée d'un monomère éthyléniquement insaturé hydrosoluble, de 0,01 à 5 % en poids par rapport audit monomère d'un agent de réticulation hydrosoluble et d'un initiateur de polymérisation hydrosoluble, ladite solution aqueuse contenant de 30 % en poids jusqu'à la concentration saturante dudit monomère, dans un dispersant (2) liquide à une température supérieure à 40 °C, choisi parmi les hydrocarbures ayant de 6 à 10 atomes de carbone et les hydrocarbures aromatiques halogénés, et contenant de 0,05 à 10 % en poids par rapport audit dispersant d'un agent tensio-actif choisi parmi les éthers et esters de cellulose, de manière à former une suspension eau dans l'huile telle que le rapport en volume du dispersant (2) à la solution aqueuse (1) soit compris entre 1 et 5, puis
- polymérisation dudit monomère en suspension, à une température supérieure à 40 °C, en présence de l'agent de réticulation et de l'initiateur.

Par ailleurs le brevet US-A-4.497.930 décrit un procédé comprenant (a) la polymérisation d'un acide carboxylique hydrophile ou son sel, dissous dans l'eau, pour obtenir un polymère hydrophile hydraté, puis (b) la déshydratation du polymère obtenu jusqu'à ce que sa teneur en eau soit comprise entre 10 et 40 % en poids, puis (c) l'addition au polymère déshydraté d'une quantité efficace d'un agent de réticulation possédant au moins 2 groupes fonctionnels. Ce procédé présente l'inconvénient de la complexité liée à la présence de trois étapes successives dont l'une, l'étape (b), est généralement longue et coûteuse en énergie. D'après les exemples fournis dans ce document, l'étape (b) est généralement conduite sous forme d'une distillation azéotropique, la proportion d'eau dans l'azéotrope formé étant de l'ordre de 7 % en poids seulement ; comme il est bien connu de l'homme de l'art, la durée d'une telle distillation est au moins égale à 3 heures. Alternativement l'étape (b) peut être réalisée au moyen d'un sécheur à air chaud, la durée de cette méthode étant (de manière connue) au moins égale à 1 heure. Compte tenu des indications de durée figurant aux exemples de ce brevet pour les étapes (a) et (c), la durée totale du processus est donc comprise au moins entre 5 et 8 heures. D'autre part on constate, en reproduisant le procédé décrit par ce

document, que les polymères obtenus possèdent une force de gel (module élastique) médiocre et de médiocres capacité et vitesse d'absorption d'eau sous contrainte.

Le brevet GB-A-2.088.392 décrit une méthode de production d'hydrogels comprenant la polymérisation en suspension eau-dans-huile d'un monomère acide carboxylique insaturé et/ou son sel alcalin en présence d'un solvant organique, d'un polymère carboxylique (agissant en tant qu'agent dispersant) ayant une affinité pour ledit solvant et le cas échéant d'un agent de réticulation. Par ailleurs les données figurant aux exemples montrent des rapports en poids de la phase huileuse à la phase aqueuse voisins de 3. Enfin, dans le cas de l'acide acrylique, les polymères obtenus en présence de réticulant ont un diamètre moyen de particules compris entre 170 µm et 700 µm.

Ainsi aucun des procédés proposés dans l'état de la technique n' est parvenu à produire des polymères présentant simultanément l'ensemble des propriétés souhaitées, énoncées précédemment, concernant l'absorption et la rétention d'eau tout en respectant l'impératif d'un prix de fabrication peu élevé. A ce dernier sujet on peut considérer comme significatifs des facteurs tels que le rapport de la phase huile à la phase aqueuse et la proportion d'agent tensio-actif utilisé.

Par ailleurs il est connu, dans le domaine de la fabrication de polymères hydrosolubles, d'avoir recours à une technique de polymérisation en émulsion avec de faibles rapports en poids de la phase huileuse à la phase aqueuse. En particulier le brevet allemand n° 1.123.110 décrit un procédé de polymérisation de monomères hydrosolubles pour préparer des polymères hydrosolubles, caractérisé en ce que l'on soumet à l'action d'initiateurs de polymérisation connus en soi une émulsion contenant au moins un émulgateur ionique et/ou non ionique, dans laquelle se trouve une phase comprenant la solution aqueuse desdits monomères ainsi que de 1 à 15% en poids d'un milieu solvant organique soluble dans l'eau, de manière à précipiter un polymère en poudre. De manière préférée, le rapport en poids de la phase huileuse à la phase aqueuse est compris entre 0,5 et 4. D'autre part le brevet américain n° 4.070.321 décrit un procédé de préparation d'émulsions eau-dans-huile de polymères hydrosolubles d'acides carboxyliques vinyliques de haut poids moléculaire; dans les exemples de ce document, le rapport en poids de la phase huileuse à la phase aqueuse est égal à 0,37. Enfin le brevet américain n° 2.982.749 décrit un procédé de fabrication de (co)polymères de monomères hydrosolubles par formation d'une suspension eau-dans-l'huile d'une solution aqueuse contenant de 5 à 80% en poids d'un tel monomère dans un milieu dispersant organique hydrophobe contenant un agent de suspension ayant une faible HLB, le ratio en poids solution aqueuse/milieu dispersant étant compris entre 5/95 et 75/25, puis polymérisation thermique en présence d'un initiateur de manière à obtenir le polymère sous forme de granulés; dans le cas de l'acide acrylique, les particules de polymère ont un diamètre variant de 35 à 100 µm.

Les trois documents analysés ci-dessus ont en commun le fait de ne pas mettre en oeuvre d'agent de réticulation, d'employer des proportions d' émulgateur relativement élevées et de conduire à la formation de polymères totalement solubles dans l'eau destinés à des usages industriels tels que agents de floculation, agents épaississants ou bien liants pour compositions de revêtement. De tels polymères sont absolument inutilisables pour les produits d'hygiène et de santé mentionnés en début de la présente description et constituant le but de la présente invention.

En vue de résoudre les différents problèmes évoqués ci-dessus tant du point de vue du procédé de fabrication de polymères capables d'absorber l'eau que du point de vue des propriétés des produits obtenus, la demanderesse a mis au point un procédé de préparation d'un polymère en poudre ayant une capacité d'absorption et de rétention d'eau élevée, consistant à disperser et à mettre en suspension une phase aqueuse (1) constituée d'une solution aqueuse d'au moins un monomère hydrosoluble éthyléniquement insaturé, contenant une quantité efficace d'au moins un agent de réticulation soluble dans ladite phase (1), dans une phase huileuse (2) constituée d'au moins un hydrocarbure aliphatique ou alicyclique ayant au moins 6 atomes de carbone et contenant une quantité efficace d'au moins un agent tensio-actif, puis à polymériser ledit monomère hydrosoluble éthyléniquement insaturé en présence d'au moins un générateur de radicaux libres, caractérisé en ce que l'agent tensio-actif est choisi de manièreà pouvoir effectuer la polymérisation avec un rapport en poids de la phase (2) à la phase (1) inférieur ou égal à 0,75, et appartient à la famille d'agents tensio-actifs telle que définie ci-après. Ainsi l'un des traits essentiels de la présente invention réside dans la découverte surprenante que, moyennant un choix judicieux de l'agent tensio-actif dans la phase huileuse, les propriétés du polymère en poudre obtenu peuvent être améliorées, tout en perfectionnant les caractéristiques économiques du procédé par un abaissement significatif du rapport pondéral de la phase (2) à la phase (1). Pour tenir compte des documents antérieurs relatifs à la fabrication de polymères hydrosolubles, un autre mode d'expression de la présente invention consiste en un procédé de préparation d'un polymère en poudre ayant une capacité d'absorption et de rétention d'eau élevée, consistant à disperser et à mettre en suspension une phase aqueuse (1) constituée d'une solution aqueuse d'au moins un monomère hydosoluble éthylèniquement insaturé, dans une phase huileuse (2) constituée

d'au moins un hydrocarbure aliphatique ou alicyclique ayant au moins 6 atomes de carbone et contenant une quantité efficace d'au moins un agent tensio-actif, le rapport en poids de la phase (2) à la phase (1) étant inférieur ou égal à 0,75, puis à polymériser ledit monomère hydosoluble éthyléniquement insaturé en présence d'au moins un générateur de radicaux libres, caractérisé en ce que en vue de préparer un polymère ayant une capacité d'absorption et de rétention d' eau élevée, la phase aqueuse (1) contient une quantité efficace d'au moins un agent de réticulation soluble dans ladite phase (1). Dans le cadre de la présente invention, le monomère hydrosoluble éthylèniquement insaturé est de préférence un monomère porteur de fonction acide tel qu'un acide ou anhydride d'acide carboxylique éthyléniquement insaturé ou un acide sulfonique éthylèniquement insaturé ou leur mélange. De tels monomères incluent l'acide acrylique, l'acide méthacrylique, l'acide éthacrylique, l'acide alpha-chloroacrylique, l'acide alpha-cyanoacrylique, l'acide crotonique, l'acide alpha-phénylacrylique, l'a cide béta-acryloxypropionique, l'acide sorbique, l'acide alpha-chlorosorbique, l'acide angélique, l'acide cinnamique, l'acide p-chlorocinnamique, l'acide béta-styrylacrylique, l'acide itaconique, l'acide citraconique, l'acide mésaconique, l'acide glutaconique, l'acide maléique, l'acide fumarique, l'anhydride maléique, l'acide vinylsulfonique, l' acide allylsulfonique, l'acide vinyltoluènesulfonique, l'acide styrènesulfonique, l'acide 2-hydroxy-3-acryloxypropylsulfonique, l'acide 2-hydroxy-3-méthacryloxypropylsulfonique et l'acide 2-acrylamido-2-méthylpropanesulfonique. Sont plus particulièrement préférés l'acide acrylique le cas échéant en mélange avec une faible proportion d'acide méthacrylique. Pour l'efficacité du procédé selon l'invention il est hautement souhaitable que ledit monomère hydrosoluble éthyléniquement insaturé soit au moins partiellement neutralisé, de préférence à raison d'au moins 50% en moles, au moyen d'une base telle que la soude, la potasse, l'ammoniaque, l'hydroxyde de lithium, etc. La solution aqueuse faisant partie de la phase (1) comprendra donc le monomère acide éthyléniquement insaturé, tel que par exemple l'acide acrylique, en mélange avec le sel alcalin ou d'ammonium correspondant de préférence en proportion majoritaire. Comme il est déjà connu notamment par le brevet FR-A-2.367.083 mentionné précédemment, la concentration du monomère hydrosoluble éthyléniquement insaturé dans la solution aqueuse de la phase (1) est de préférence supérieure ou égale à 40 % en poids.

Bien entendu un agent tensio-actif répondant au critère de choix précité (à savoir permettre d'effectuer la polymérisation avec un rapport en poids de la phase (2) à la phase (1) inférieur ou égal à 0,75) pourra aussi être utilisé pour effectuer la polymérisation avec un rapport en poids de la phase (2) à la phase (1) supérieur à 0,75 et pouvant atteindre jusqu'à 5 environ. Une telle utilisation, pour autant que ledit agent tensio-actif n'ait pas déjà été proposé pour la fabrication de polymères en poudre ayant une capacité d'absorption et de rétention d'eau élevée, entre également dans le cadre de la présente invention.

Le choix d'un agent tensio-actif répondant au critère énoncé précédemment s'est révélé difficile. En effet aucun des agents tensio-actifs connus pour la fabrication de tels polymères en poudre n'a pu être utilisé en effectuant la polymérisation avec un rapport en poids de la phase (2) à la phase (1) inférieur à 1. Ceci est vrai tant des esters d'acide gras de sorbitan ayant une valeur HLB de 3 à 6 décrits dans le brevet FR-A-2.367.083 que des esters et éthers d'acide gras de sorbitan ou de sorbitol ayant une valeur HLB de 8 à 12 décrits dans le brevet EP-B-0.036.463 et que des esters et éthers de cellulose décrits dans le brevet US-A-4.446.261.

Une famille d'agents tensio-actifs répondant au critère de choix de la présente invention consiste en des copolymères comprenant au moins deux composants polymériques dérivés d'acides monocarboxyliques complexes liposolubles et un autre composant polymérique résidu d'un composé hydrosoluble contenant des motifs polyoxyalkylène, lesdits copolymères étant repré sentés par la formule générale $(A\text{-}COO)_m\text{-}B$ dans laquelle m est un nombre entier au moins égal à 2, chaque composant polymérique A possède un poids moléculaire au moins égal à 500 et est le résidu d'un acide monocarboxylique complexe liposoluble de formule générale

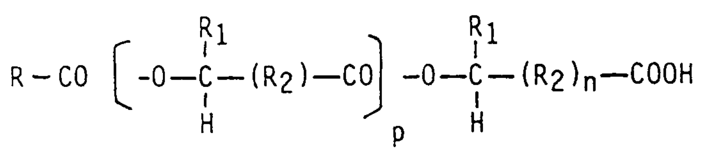

(I)

dans laquelle:

- . R est l'hydrogène ou bien un groupe hydrocarboné monovalent ou substitué,
- . $R_1$ est l'hydrogène ou bien un groupe hydrocarboné monovalent ayant de 1 à 24 atomes de carbone,
- . $R_2$ est un groupe hydrocarboné divalent en $C_1$ à $C_{24}$ ;
- . n est égal à 0 ou bien 1,

. p est un nombre entier allant de 0 jusqu'à 200 ;
chaque composant polymérique B a un poids moléculaire au moins égal à 500 et, dans le cas où m est égal à 2, est le résidu bivalent d'un polyalkylène glycol hydrosoluble de formule générale

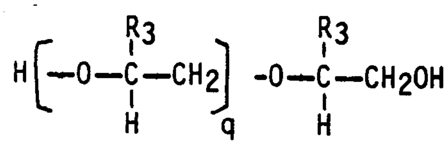 (II)

dans laquelle :

. $R_3$ est l'hydrogène ou bien un groupe alkyle ayant de 1 à 3 atomes de carbone,

. q est un nombre entier allant de 10 jusqu'à 500,

ou bien, dans le cas où m est supérieur à 2, est le résidu de valence m d'un polyéther polyol hydrosoluble de formule générale

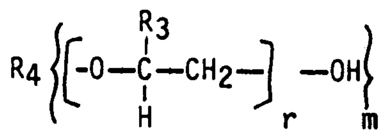 (III)

dans laquelle :

. $R_3$ et m ont la signification précédente,

. r est un nombre entier allant de 0 jusqu'à 500, sous la condition que le nombre total d'unités

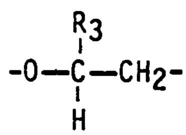

dans la molécule soit au moins égal à 10,

. $R_4$ est le résidu d'un composé organique contenant m atomes d'hydrogène réactifs avec un oxyde d'alkylène.

Un tel agent tensio-actif a déjà été décrit notamment dans le brevet US-A-4.203.877. Lorsqu'il est mis en oeuvre selon l'invention:

- sa valeur HLB peut être choisie dans une gamme allant de 2 jusqu'à 16, de préférence entre 4 et 10, en fonction de sa constitution chimique.
- il est généralement efficace dans un rapport pondéral, relativement à la phase huileuse (2), supérieur ou égal à environ $2.10^{-4}$. Pour une efficacité optimale, il n'est généralement pas nécessaire de l'utiliser dans un rapport pondéral, relativement à la phase huileuse (2), supérieur à $2.10^{-2}$.

Les agents tensio-actifs décrits ci-dessus peuvent dans une certaine mesure être utilisés, dans le procédé selon l'invention, en mélange avec de faibles proportions d'autres agents tensio-actifs non ioniques.

Comme agent de réticulation soluble dans la phase (1) et utilisable dans le cadre de la présente invention on peut citer par exemple:

1) des composés ayant au moins deux doubles liaisons polymérisables et,

2) des composés ayant au moins une double liaison polymérisable et au moins un groupe fonctionnel réactif avec le monomère hydrosoluble éthyléniquement insaturé.

3) des composés ayant au moins deux groupes fonctionnels réactifs avec le monomère hydrosoluble éthyléniquement insaturé.

Des exemples des composés répertoriés en premièrement ci-dessus, ayant au moins deux doubles liaisons polymérisables, sont:

a) les composés di- ou polyvinyliques, tels que notamment le divinylbenzène, le divinyltoluène, le divinylxylène, l'éther divinylique, la divinylcétone et le trivinylbenzène,

b) les di- ou polyesters d'acides mono- ou polycarboxyliques non saturés avec des polyols, tels que les esters des acides di- ou tri(méth)acryliques avec des polyols (tels que l'éthylène glycol, le triméthylolpropane, le glycérol, les polyoxyéthylèneglycols, les polyoxypropylèneglycols, etc.), les polyesters non saturés (que l'on peut obtenir par réaction de l'un quelconque des polyols précités avec un acide non saturé tel que l'acide maléique), les esters d'acide di- ou tri-(meth)acrylique que l'on peut obtenir par réaction d'un polyépoxide avec l'acide (méth)acrylique),

c) les bis (méth)acrylamides tels que le N,N-méthylène-bis-acrylamide et le glyoxal-bis-acrylamide,

d) les esters carbamyliques que l'on peut obtenir en faisant réagir des polyisocyanates (tels que le toluène diisocyanate, l'hexaméthylène diisocyanate, le 4,4'-diphénylméthanediisocyanate, etc. et les prépolymères contenant un groupe NCO obtenus en faisant réagir un tel diisocyanate avec des composés contenant des atomes d'hydrogène actifs) avec des monomères contenant des groupes hydroxyles. De tels esters sont notamment ceux des acides di(méth)acryliques que l'on peut obtenir en faisant réagir les diisocyanates précités avec le (méth)acrylate d'hydroxyéthyle,

e) les éthers di- ou poly(méth)allyliques de polyols tels que les alkylènes glycols, le glycérol, les polyalkylèneglycols, les polyoxyalkylènepolyols, les hydrates de carbones etc), tels que l'éther diallylique du polyéthylène glycol,l'amidon allylé et la cellulose allylée,

f) les esters di- ou polyallyliques d'acides polycarboxyliques tels que le phthalate de diallyle, l'adipate de diallyle etc., et

g) les esters d'acides mono- ou polycarboxyliques non saturés avec des éthers mono(méth)allyliques de polyols, tels que l'ester de l'acide (méth)acrylique avec l'éther monoallylique du polyéthylène glycol.

Les composés du type deuxièmement précité ayant au moins une double liaison polymérisable et au moins un groupe fontionnel réactif avec au moins l'un des monomères sont les composés éthyléniquement insaturés contenant au moins un groupe réactif avec les groupes carboxyle, anhydride carboxylique, hydroxyle, amine ou amide. Des exemples de ces composés sont la N-méthylol(méth)acrylamide, le (méth)-acrylate de glycidyle, etc.

Parmi les composés du type troisièmement précité figurent notam-ment le glyoxal, les polyols tels que l'éthylène glycol, les polyamines tels que les alkylène diamines, les polyalkylène polyamines, les polyépoxydes, les éthers de di- ou polyglycidyle, etc.

Par quantité efficace d'agent de réticulation on entend, au sens de la présente invention, une quantité comprise entre environ 0,04 % et 2 % en poids par rapport au monomère hydrosoluble éthyléniquement insaturé.

Tout moyen capable de générer des radicaux libres pourra être utilisé dans le cadre du procédé selon l'invention. Il pourra s'agir notamment de microondes, de rayonnements béta, gamma ou ultraviolet, ou encore d'initiateurs chimiques hydrosolubles. Dans ce dernier cas l'initiateur de polymérisation peut être notamment choisi parmi les persulfates, les peroxydes, les hydroperoxydes et les composés diazoïques (tel que le 2,2' azobis(2-amidinopropane)dihydrochlorure) ; lorsqu'un persulfate de métal alcalin est choisi, il peut être utilisé en combinaison avec un réducteur choisi parmi les polyhydrophénols, le sulfite et le bisulfite de sodium, l'acide L-ascorbique, le diméthylaminopropionitrile, les diazomercaptans, les ferricyanures et le sulfate ferreux. L'initiateur et, le cas échéant, le réducteur sont utilisables à raison de 0,05 à 2 % chacun en poids par rapport au monomère hydrosoluble éthyléniquement insaturé.

Le plus souvent la réaction de polymérisation sera effectuée sous pression atmosphérique. La température et la durée de polymérisation seront choisies l'une en fonction de l'autre, la durée étant d'autant plus longue que la température est plus basse et vice-versa. Lorsqu'on utilise un initiateur chimique hydrosoluble, la durée de la réaction est généralement au moins égale à 10 minutes et de préférence au plus égale à 200 minutes. A l' issue de cette durée, la polymérisation est totale et les monomères résiduels sont éliminés pour la plus grande partie. Le polymère en poudre est ensuite récupéré de préférence par précipitation dans un alcool léger tel que notamment méthanol, éthanol ou isopropanol. Une fraction complémentaire des monomères résiduels est à nouveau éliminée à ce stade. Lorsqu'on utilise un initiateur chimique hydrosoluble, la réaction de polymérisation est de préférence initiée à une température au moins égale à 20 °C environ et au plus égale à la température d'ébullition de l'azéotrope formé entre l'eau et l'hydrocarbure de la phase (2), par exemple au plus égale à 90 °C environ, puis la température est maintenue dans cette gamme pendant toute la durée de la réaction telle que précisée précédemment. Lorsque les radicaux libres sont générés par un rayonnement, la température peut être de l'ordre de 10 °C à 50 °C seulement.

Les polymères en poudre obtenus par le procédé selon la présente invention possèdent des propriétés d'usage tout à fait particulières qui répondent de manière optimale aux besoins des utilisateurs, notamment dans le domaine de l'hygiène et de la santé. En effet tout en possédant une vitesse et une capacité d'absorption et de rétention d'eau, en particulier d'eau saline, élevées comme les produits obtenus par les procédés antérieurement connus, ils se distinguent de ces derniers par au moins l'une des deux propriétés suivantes :

- le module élastique du gel obtenu après adsorption d'eau saline physiologique par le polymère. Cette propriété, dont la méthode de mesure est précisée dans les exemples ci-après, est Importante pour apprécier la consistance, dure ou molle, du gel. Qualitativement parlant, un gel de module élastique élevé (consistance dure) présente un aspect "sec" au toucher, tandis qu'un gel de module élastique faible (consistance molle) présente un aspect "coulant" au toucher.
- la capacité de succion capillaire sous pression. Cette propriété, dont la méthode de mesure est précisée dans les exemples ci-après, permet une simulation satisfaisante des conditions auxquelles est soumise le polymère lorsqu'il est déposé dans un article textile hygiénique, tel qu'une couche pour bébé ou incontinent, et qu'il se trouve en présence d'un flux d'eau saline (sang et/ou urine) arrivant à travers les fibres de cellulose. Pour cette raison, la capacité de succion capillaire sous pression est importante pour apprécier l'aptitude du polymère à entrer dans la constitution d'articles hygiéniques capables d'absorber des fluides aqueux.
- la teneur du polymère en monomère résiduel. Cette propriété, dont la méthode de mesure est précisée dans les exemples ci-après, est importante du point de vue toxicologique.
- la teneur en polymère extractible. Cette propriété, dont la méthode de mesure est précisée dans les exemples ci-après, est également importante du point de vue toxicologique, notamment dans les conditions d'usage du produit les plus courantes.

Un second objet de la présente Invention consiste donc en un polymère en poudre ayant une capacité d'adsorption et de rétention d'eau élevée, à base d'au moins un monomère hydrosoluble éthyléniquement insaturé, caractérisé en ce que sa capacité de succion capillaire sous pression après 20 minutes est au moins égale à 20 ml/g, et en ce qu'il est susceptible d' être obtenu par le procédé décrit précédemment. On notera en particulier que le polymère en poudre selon l'invention peut présenter une capacité de de succion capillaire sous pression allant jusqu'à 30 ml/g environ. Un gel obtenu après absorption d'eau saline physiologique par ledit polymère possède généralement un module élastique au moins égal à 0,35 $N/cm^2$ (35.000 $dynes/cm^2$) environ et pouvant aller jusqu'à 0,6 $N/cm^2$ (60.000 $dynes/cm^2$) environ. La répartition granulométrique, observée au microscope optique ou électronique, des polymères en poudre selon l'invention fait apparaître un diamètre moyen des particules généralement compris entre 110 et 220 $\mu m$.

Les polymères en poudre selon l'invention présentent avantageusement de faibles proportions de substances extractibles par l'eau saline. Ainsi la teneur en monomères résiduels est-elle généralement comprise entre 60 et 180 ppm environ, la teneur en polymère extractible entre 4 et 10% environ.

Les polymères en poudre selon l'invention présentent en outre l' avantage considérable, notamment pour des applications dans le domaine agricole où elles sont susceptibles d'être soumises au rayonnement solaire, de conserver leurs propriétés d'absorption et de rétention d'eau après une exposition prolongée au rayonnement ultraviolet.

Enfin un troisième objet de la présente invention consiste en des articles capables d'absorber les fluides aqueux, comprenant au moins un polymère en poudre tel que décrit précédemment. De tels articles peuvent trouver des usages variés dans le domaine de l'hygiène (couches pour bébés et incontinents) en raison de leur capacité à absorber des fluides corporels tels que le sang et/ou l'urine, ainsi que dans le domaine de l'agriculture (rétention de l'eau dans les zones géographiques faiblement arrosées) et de l'industrie (agents déshydratants).

Les exemples ci-après sont donnés à titre illustratif et non limitatif de la présente invention.

## EXEMPLE 1

78 g d'acide acrylique sont neutralisés par 153 g d'une solution aqueuse contenant 22 % en poids de soude. Au cours de la neutralisation, on refroidit le mélange réactionnel pour éviter une polymérisation accidentelle de l'acide acrylique.

A la solution d'acide acrylique et acrylate de sodium ainsi obtenue, on ajoute ensuite 0,156 g de méthylène-bis-acrylamide sous agitation, puis 260 mg de persulfate d'ammonium et 7,8 mg de sel de Mohr.

Simultanément à la préparation de la phase aqueuse, 0,6 g d'un agent tensio-actif commercialisé par la société IMPERIAL CHEMICAL INDUSTRIES sous la dénomination B 261 et ayant une valeur HLB égale à 8 est dissout dans 428 g de cyclohexane en chauffant le mélange à 45 °C pendant quelques minutes. Cet agent tensio-actif est un copolymère comprenant au moins deux composants polymériques dérivés d'acides monocarboxyliques complexes liposolubles et un autre composant polymérique résidu d'un composé hydrosoluble contenant des motifs polyoxyalkylène.

La phase aqueuse est alors introduite dans la phase huileuse dans un réacteur à double enveloppe en maintenant la température à 20 °C, puis on élève la température à 65 °C. La conversion totale est atteinte en 15 minutes. On maintient ensuite le mélange réactionnel à 65 °C pendant une heure afin d'éliminer les

monomères résiduels. Le polymère est ensuite récupéré par précipitation dans du méthanol puis séché à 70 ° C dans une étuve ventilée.

Sur le polymère en poudre ainsi obtenu on mesure les propriétés suivantes :

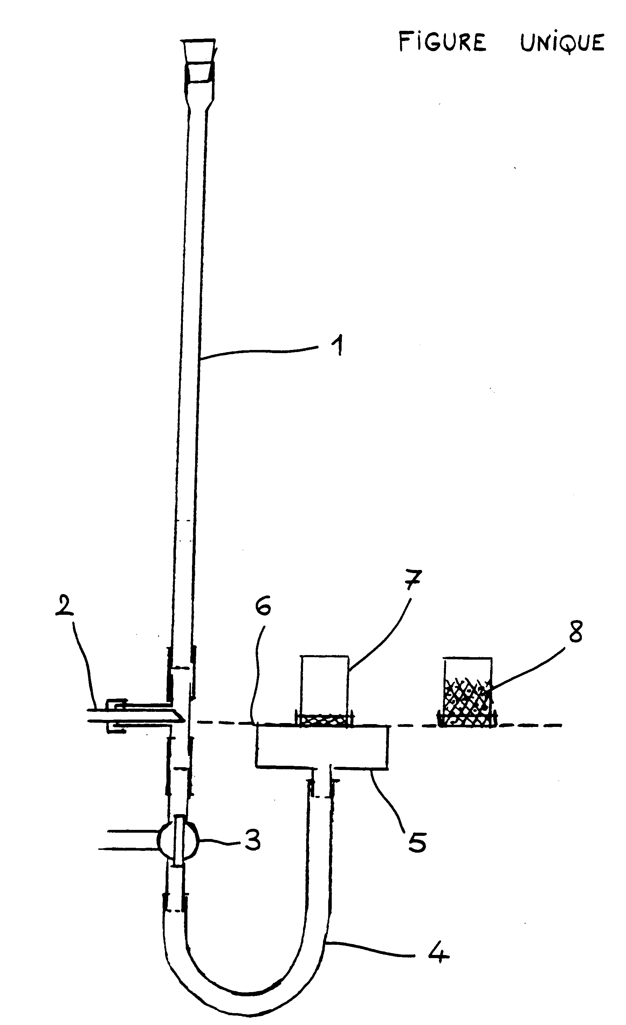

- capacité d'absorption et de rétention d'eau saline (comprenant 1% de chlorure de sodium) : exprimée en g/g, elle est déterminée selon la méthode décrite par le brevet EP-A-0.188.963 page 5 lignes 38-39.
- diamètre moyen D des particules : cette propriété est obtenue par calcul à partir de l'histogramme des tailles de particules observées au microscope optique.
- module élastique ME du gel : la méthode suivie consiste à placer 58 g d' une solution aqueuse de chlorure de sodium à 10 g/l dans un bécher de 100 ml muni d'une agitation magnétique, puis à ajouter 2 g du polymère en poudre à cette solution. L'ensemble est laissé sous agitation jusqu'à ce que, sous l'effet de l'augmentation de la viscosité, la surface de la solution dans le bécher devienne horizontale (disparition du vortex). On mesure alors le module élastique du gel, exprimé en $N/cm^2$, au moyen d'un pénétromètre commercialisé par la société ELECTRIC CORP.OF JAPAN sous la dénomination M-302.
- la capacité de succion capillaire sous pression SC : la méthode suivie répond au principe qui sera maintenant décrit avec référence au schéma représenté sur la figure unique en annexe. Une burette remplie de liquide, bouchée à son extrémité supérieure et munie d'une arrivée d'air à sa base, est reliée à une cellule recouverte d'une grille métallique. La grille métallique est mise de niveau avec l'arrivée d'air de la burette. Le polymère en poudre, posé sur la grille, en absorbant du liquide crée un appel d'air dans la burette maintenant une pression hydrostatique nulle au niveau de la grille support. De façon plus précise l'appareillage comprend une burette graduée (1) de 50 ml munie d'une arrivée d'air, un tube d'arrivée d'air (2) de 4 mm de diamètre intérieur, un robinet à 3 voies (3) relié à un réservoir (non représenté sur la figure), un tube souple (4) de 10 mm de diamètre, une cellule (5) en polychlorure de vinyle (PVC) transparent de 14 cm de diamètre avec un orifice de 1,2 cm de diamètre et recouverte d'une grille métallique (6) de maillage de 1 mm, et un tube (7) en PVC transparent de 10 cm de hauteur et de 5 cm de diamètre intérieur. On opère comme suit : la burette (1) est d'abord complètement remplie par le liquide d'essai, à savoir une solution aqueuse de chlorure de sodium à 10 g/l ; on prépare ensuite l'échantillon en fermant une extrémité du tube (7) par une double épaisseur de non-tissé, sur lequel on répartit uniformément 0,5 g de polymère en poudre. On recouvre ensuite le polymère en poudre d'un disque de non-tissé et d'une grille métallique de 5 cm de diamètre, par dessus laquelle on pose 400 grammes de billes métalliques (8) d'environ 5 mm de diamètre, de manière à créer une pression de 2000 Pa. La capacité de succion capillaire SC, s'exprime en ml/g comme le volume de liquide absorbé par gramme de polymère en poudre au bout de 20 minutes.
- la teneur en polymère extractible PE : la méthode suivie consiste à placer 200 g de solution saline à 9 g NaCl par litre dans un bécher de 500 ml et régler l'agitation à environ 300 tr/mn, verser alors 1 g de polymère en poudre dans le bécher, laisser l'ensemble sus agitation pendant trois heures, filtrer ensuite la solution sur un tamis en maille métallique de diamètre 10 cm et d'ouverture de maille 75 µm et recueillir l'éluat dans un cristallisoir de 500 ml,laisser égoutter pendant 30 minutes et peser alors l'éluat (masse = $m_1$), placer le cristallisoir dans une étuve ventilée (75 ° C), pendant environ 15 heures, terminer le séchage dans une étuve aérée (140 ° C), pendant environ 4 heures, mettre le cristallisoir dans un dissicateur pour laisser revenir dans le cristallisoir (masse = $m_2$). La teneur en polymère extractible exprimée en pour-cent par rapport au polymère est donnée par la formule:

$$PE = \frac{\left(m_2 - m_1 \times \frac{0,9}{100}\right)}{1,0} \times 100$$

- la teneur en monomère résiduel MR : la méthode suivie consiste à ajouter 2 g de polymére en poudre à 500 g d'une solution de chlorure de sodium à 0,9%. Après 3 heures d'agitation, la solution est filtrée sur une toile métallique de 200 mesh (0,074mm). Le filtrat est injecté dans un chromatographe liquide haute pression. La teneur en monomère (acide acrylique) résiduel, exprimée en ppm, est obtenue en mesurant la surface du pic du chromatogramme par rapport aux pics de référence

obtenus à partir de solutions étalons à 100 ppm, 10 ppm et 1 ppm en acide acrylique.
Les résultats des mesures effectuées figurent au tableau ci-après.

**EXEMPLE 2**

On reproduit le mode opératoire de l'exemple 1 en remplaçant, dans la phase huileuse, le cyclohexane par l'heptane. Les propriétés du polymère obtenu sont reportées dans le tableau ci-après.

**EXEMPLE 3**

On reproduit le mode opératoire de l'exemple 1 en remplaçant en la phase huileuse, le cyclohexane par l'heptane. Les propriétés du polymère obtenu sont reportées dans le tableau ci-après.

**EXEMPLE 3**

On reproduit le mode opératoire de l'exemple 1 en remplaçant en tant qu'agent de réticulation, la méthylène-bis-acrylamide par la glyoxal-bis-acrylamide. Les propriétés du polymère obtenu sont reportées dans le tableau ci-après.

**EXEMPLES 4 et 5**

On reproduit le mode opératoire de l'exemple 1 en diminuant la quantité de méthylène-bis-acrylamide : celle-ci est égale respectivement à 0,039 g (exemple 4) et 0,078 g (exemple 5). Les propriétés des polymères obtenus sont reportées dans le tableau ci-après.

**EXEMPLES 6 à 8**

On reproduit le mode opératoire de l'exemple 1 en diminuant la quantité de cyclohexane dans la phase huileuse : celle-ci est égale respectivement à 128 g (exemple 6), 162 g (exemple 7) et 78,5 g (exemple 8). Les propriétés des polymères obtenus sont reportées dans le tableau ci-après.

**EXEMPLE 9**

On reproduit le mode opératoire de l'exemple 7 en remplaçant l'agent tensio-actif par un autre produit, de constitution similaire, commercialisé par la société IMPERIAL CHEMICAL INDUSTRIES et ayant une valeur HLB égale à 5,5. Les propriétés du polymère obtenu sont reportées au tableau ci-après.

**EXEMPLE 10 (COMPARATIF)**

On reproduit le mode opératoire de l'exemple 1 en utilisant comme agent tensio-actif 2,2 g de monostéarate de sorbitan commercialisé sous la dénomination MONTANE 60 et ayant une valeur HLB égale à 4,7. Les propriétés du polymère obtenu sont reportées au tableau ci-après.

**EXEMPLE 11 (COMPARATIF)**

On reproduit le mode opératoire de l'exemple 6 en utilisant l'agent tensio-actif de l'exemple 10. Lorsque la température du réacteur atteint 60 °C, la polymérisation devient très violente, la suspension se déstabilise et il en résulte une prise en masse de l'ensemble des réactifs dans le réacteur. Aucun polymère en poudre ne peut donc être isolé.

**TABLEAU**

| Exemple | AE | D | ME | SC | PE | MR |
|---|---|---|---|---|---|---|
| 1 | 50 | 120 | 0,50 | 26 | 9 | 120 |
| 2 | 60 | 130 | n.d. | 26 | 8 | 80 |
| 3 | 50 | 130 | 0,48 | 27 | 8 | 140 |
| 4 | 60 | 140 | 0,37 | 30 | 10 | 180 |
| 5 | 40 | 180 | 0,57 | 26 | 5 | 70 |
| 6 | 48 | 180 | 0,50 | 26 | n.d. | n.d. |
| 7 | 50 | 190 | 0,47 | 28 | n.d. | n.d. |
| 8 | 47 | 200 | 0,51 | 26 | 7 | 110 |
| 9 | 50 | 170 | n.d. | 27 | n.d. | n.d. |
| 10 | n.d. | 90 | 0,31 | 17 | n.d. | n.d. |

n.d. : non déterminé

**Revendications**

**Revendications pour les Etats contractants suivants : DE, GB, FR, IT, NL, SE, LI, CH, BE, LU, GR**

**1.** Procédé de préparation d'un polymère en poudre ayant une capacité d'absorption et de rétention d'eau élevée, consistant :

- à disperser et à mettre en suspension une phase aqueuse (1) constituée d'une solution aqueuse d'au moins un monomère hydrosoluble éthyléniquement insaturé, contenant une quantité efficace pour assurer la réticulation du polymère visé d'au moins un agent de réticulation soluble dans ladite phase (1), dans une phase huileuse (2) constituée d'au moins un hydrocarbure aliphatique ou alicyclique ayant au moins 6 atomes de carbone et contenant une quantité efficace d'au moins un agent tensio-actif, puis ;
- à polymériser ledit monomère hydrosoluble éthyléniquement insaturé en présence d'au moins un générateur de radicaux libres,

caractérisé en ce que l'agent tensio-actif est un copolymère comprenant au moins deux composants polymériques dérivés d'acides monocarboxyliques complexes liposolubles et un autre composant polymérique résidu d'un composé hydrosoluble contenant des motifs polyoxyalkylène, lesdits copolymères étant représentés par la formule générale :

$(A\text{-}COO)_m\text{-}B$

dans laquelle :

- m est un nombre entier au moins égal à 2 ;
- chaque composant polymérique A possède une masse moléculaire au moins égale à 500 et est le résidu d'un acide monocarboxylique complexe liposoluble de formule générale :

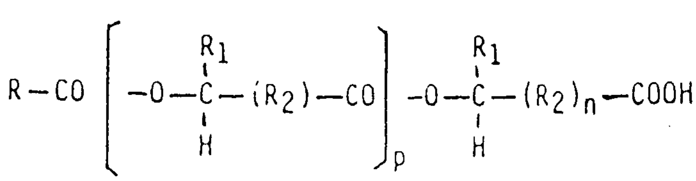

(I)

dans laquelle :
- R est l'hydrogène ou bien un groupe hydrocarboné monovalent ou substitué,
- $R_1$ est l'hydrogène ou bien un groupe hydrocarboné monovalent ayant de 1 à 24 atomes de carbone,
- $R_2$ est un groupe hydrocarboné divalent en $C_1$ à $C_{24}$
- n est égal à 0 ou bien 1,
- p est un nombre entier allant de 0 jusqu'à 200 ; et

- chaque composant polymérique B a une masse moléculaire au moins égale à 500 et, dans le cas où m est égal à 2, est le résidu bivalent d'un polyalkylène glycol hydrosoluble de formule générale :

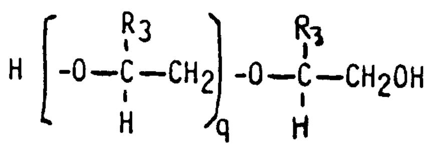 (II)

dans laquelle :
- $R_3$ est l'hydrogène ou bien un groupe alkyle ayant de 1 à 3 atomes de carbone,
- q est un nombre entier allant de 10 jusqu'à 500,

ou bien, dans le cas où m est supérieur à 2, est le résidu de valence m d'un polyéther polyol hydrosoluble de formule générale

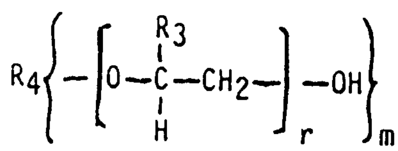 (III)

dans laquelle :
- $R_3$ et m ont la signification précédente,
- r est un nombre entier allant de 0 jusqu'à 500, sous la condition que le nombre total d'unités

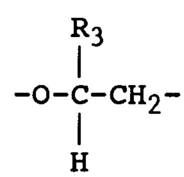

dans la molécule soit au moins égal à 10,
- $R_4$ est le résidu d'un composé organique contenant m atomes d'hydrogène réactifs avec un oxyde d'alkylène.

**2.** Procédé selon la revendication 1, caractérisé en ce que le rapport en poids de la phase (2) à la phase (1) est inférieur ou égal à 5.

**3.** Procédé selon la revendication 1, caractérisé en ce que le rapport en poids de la phase (2) à la phase (1) est inférieur ou égal à 0,75.

**4.** Procédé selon l'une des revendications 1 à 3, caractérisé en ce que le monomère hydrosoluble éthyléniquement insaturé est choisi parmi l'acide acrylique et ses sels alcalins ou d'ammonium.

**5.** Procédé selon la revendication 4, caractérisé en ce que le monomère hydrosoluble éthyléniquement insaturé est un mélange d'une proportion majoritaire d'acrylate alcalin ou d'ammonium et d'une

proportion minoritaire d'acide acrylique.

6. Procédé selon l'une des revendications 1 à 5, caractérisé en ce que la concentration du monomère hydrosoluble éthyléniquement insaturé dans la solution aqueuse de la phase (1) est supérieure ou égale à 40% en poids.

7. Procédé selon l'une des revendications 1 à 6, caractérisé en ce que la valeur HLB de l'agent tensio-actif est comprise entre 2 et 16.

8. Procédé selon l'une des revendications 1 à 7, caractérisé en ce que l'agent tensio-actif est mis en oeuvre dans un rapport pondéral, relativement à la phase (2), supérieur ou égal à $2 \times 10^{-4}$.

9. Procédé selon l'une des revendications 1 à 8, caractérisé en ce que l'agent tensio-actif est mis en oeuvre dans un rapport pondéral, relativement à la phase (2), non supérieur à $2 \times 10^{-2}$.

10. Procédé selon l'une des revendications 1 à 9, caractérisé en ce que l'agent de réticulation est choisi parmi des composés ayant au moins deux liaisons polymérisables et des composés ayant au moins une double liaison polymérisable et au moins un groupe fonctionnel réactif avec le monomère hydrosoluble éthyléniquement insaturé.

11. Procédé selon l'une des revendications 1 à 10, caractérisé en ce que la quantité d'agent de réticulation est comprise entre 0,04% et 2% en poids par rapport au monomère hydrosoluble éthyléniquement insaturé.

12. Procédé selon l'une des revendications 1 à 11, caractérisé en ce que le générateur de radicaux libres est choisi parmi les microondes et les rayonnements bêta, gamma et ultraviolet.

13. Procédé selon la revendication 12, caractérisé en que la polymérisation est effectuée à une température comprise entre 10 ° C et 50 ° C.

14. Procédé selon l'une des revendications 1 à 13, caractérisé en ce que le générateur de radicaux libres est un initiateur chimique hydrosoluble.

15. Procédé selon la revendication 14, caractérisé en ce que ledit initiateur est choisi parmi les persulfates, les peroxydes, les hydroperoxydes et les composés diazoïques.

16. Procédé selon la revendication 15, caractérisé en ce que ledit initiateur est un persulfate de métal alcalin et en ce qu'il utilisé en combinaison avec un réducteur choisi les polyhydrophénols, le sulfite et le bisulfite de sodium, le diméthylaminopropionitrile, les diazomercaptans, les ferricyanures et le sulfate ferreux.

17. Procédé selon la revendication 16, caractérisé en ce que ledit réducteur est utilisé à raison de 0,05 à 2% en poids par rapport au monomère hydrosoluble éthyléniquement insaturé.

18. Procédé selon l'une des revendications 14 à 17, caractérisé en ce que l'initiateur chimique hydrosoluble est utilisé à raison de 0,05 à 2% en poids par rapport au monomère hydrosoluble éthyléniquement insaturé.

19. Procédé selon l'une des revendications 14 à 18, caractérisé en ce que la polymérisation est effectuée à une température comprise entre 20 ° C et la température d'ébullition de l'azéotrope formé entre l'eau et l'hydrocarbure de la phase (2).

20. Procédé selon l'une des revendications 14 à 19, Caractérisé en ce que la durée de la réaction est comprise entre 10 et 200 minutes.

21. Polymère en poudre ayant une capacité d'absorption et de rétention d'eau élevée, à base d'au moins un monomère hydrosoluble éthyléniquement insaturé, caractérisé en ce que sa capacité de succion capillaire sous pression après 20 minutes est au moins égale à 20 ml/g, et en ce qu'il est susceptible

d'être obtenu par le procédé selon l'une des revendications 1 à 20.

**22.** Polymère en poudre selon la revendication 21, caractérisé en ce que le diamètre moyen des particules est compris entre 110 et 220 $\mu$m.

**23.** Polymère en poudre selon l'une des revendications 21 et 22, caractérisé en ce qu'un gel obtenu après absorption d'eau saline physiologique par ledit polymère possède un module élastique au moins égal à 0,35 N/cm$^2$ (35.000 dynes/cm$^2$).

**24.** Articles capables d'absorber les fluides aqueux, caractérisés en ce qu'ils comprennent au moins un polymère en poudre selon l'une des revendications 21 à 23.

**Revendications pour les Etats contractants suivants : AT, ES**

**1.** Procédé de préparation d'un polymère en poudre ayant une capacité d'absorption et de rétention d'eau élevée, consistant :

- à disperser et à mettre en suspension une phase aqueuse (1) constituée d'une solution aqueuse d'au moins un monomère hydrosoluble éthyléniquement insaturé, contenant une quantité efficace pour assurer la réticulation du polymère visé d'au moins un agent de réticulation soluble dans ladite phase (1), dans une phase huileuse (2) constituée d'au moins un hydrocarbure aliphatique ou alicyclique ayant au moins 6 atomes de carbone et contenant une quantité efficace d'au moins un agent tensio-actif, puis ;
- à polymériser ledit monomère hydrosoluble éthyléniquement insaturé en présence d'au moins un générateur de radicaux libres,

caractérisé en ce que l'agent tensio-actif est un copolymère comprenant au moins deux composants polymériques dérivés d'acides monocarboxyliques complexes liposolubles et un autre composant polymérique résidu d'un composé hydrosoluble contenant des motifs polyoxyalkylène, lesdits copolymères étant représentés par la formule générale :

$(A\text{-}COO)_m\text{-}B$

dans laquelle :

- m est un nombre entier au moins égal à 2 ;
- chaque composant polymérique A possède une masse moléculaire au moins égale à 500 et est le résidu d'un acide monocarboxylique complexe liposoluble de formule générale :

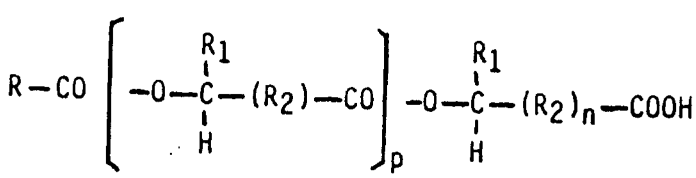

(I)

dans laquelle :

- R est l'hydrogène ou bien un groupe hydrocarboné monovalent ou substitué,
- $R_1$ est l'hydrogène ou bien un groupe hydrocarboné monovalent ayant de 1 à 24 atomes de carbone,
- $R_2$ est un groupe hydrocarboné divalent en $C_1$ à $C_{24}$
- n est égal à 0 ou bien 1,
- p est un nombre entier allant de 0 jusqu'à 200 ; et

- chaque composant polymérique B a une masse moléculaire au moins égale à 500 et, dans le cas où m est égal à 2, est le résidu bivalent d'un polyalkylène glycol hydrosoluble de formule générale :

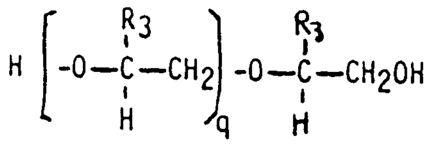 (II)

dans laquelle :

- $R_3$ est l'hydrogène ou bien un groupe alkyle ayant de 1 à 3 atomes de carbone,
- q est un nombre entier allant de 10 jusqu'à 500,

ou bien, dans le cas où m est supérieur à 2, est le résidu de valence m d'un polyéther polyol hydrosoluble de formule générale

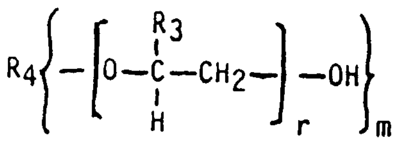 (III)

dans laquelle :

- $R_3$ et m ont la signification précédente,
- r est un nombre entier allant de 0 jusqu'à 500, sous la condition que le nombre total d'unités

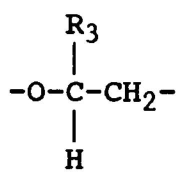

dans la molécule soit au moins égal à 10,
- $R_4$ est le résidu d'un composé organique contenant m atomes d'hydrogène réactifs avec un oxyde d'alkylène.

**2.** Procédé selon la revendication 1, caractérisé en ce que le rapport en poids de la phase (2) à la phase (1) est inférieur ou égal à 5.

**3.** Procédé selon la revendication 1, caractérisé en ce que le rapport en poids de la phase (2) à la phase (1) est inférieur ou égal à 0,75.

**4.** Procédé selon l'une des revendications 1 à 3, caractérisé en ce que le monomère hydrosoluble éthyléniquement insaturé est choisi parmi l'acide acrylique et ses sels alcalins ou d'ammonium.

**5.** Procédé selon la revendication 4, caractérisé en ce que le monomère hydrosoluble éthyléniquement insaturé est un mélange d'une proportion majoritaire d'acrylate alcalin ou d'ammonium et d'une proportion minoritaire d'acide acrylique.

**6.** Procédé selon l'une des revendications 1 à 5, caractérisé en ce que la concentration du monomère hydrosoluble éthyléniquement insaturé dans la solution aqueuse de la phase (1) est supérieure ou égale à 40% en poids.

**7.** Procédé selon l'une des revendications 1 à 6, caractérisé en ce que la valeur HLB de l'agent tensio-actif est comprise entre 2 et 16.

**8.** Procédé selon l'une des revendications 1 à 7, caractérisé en ce que l'agent tensio-actif est mis en oeuvre dans un rapport pondéral, relativement à la phase (2), supérieur ou égal à $2 \times 10^{-4}$.

**9.** Procédé selon l'une des revendications 1 à 8, caractérisé en ce que l'agent tensio-actif est mis en oeuvre dans un rapport pondéral, relativement à la phase (2), non supérieur à 2 x $10^{-2}$.

**10.** Procédé selon l'une des revendications 1 à 9, caractérisé en ce que l'agent de réticulation est choisi parmi des composés ayant au moins deux liaisons polymérisables et des composés ayant au moins une double liaison polymérisable et au moins un groupe fonctionnel réactif avec le monomère hydrosoluble éthyléniquement insaturé.

**11.** Procédé selon l'une des revendications 1 à 10, caractérisé en ce que la quantité d'agent de réticulation est comprise entre 0,04% et 2% en poids par rapport au monomère hydrosoluble éthyléniquement insaturé.

**12.** Procédé selon l'une des revendications 1 à 11, caractérisé en ce que le générateur de radicaux libres est choisi parmi les microondes et les rayonnements bêta, gamma et ultraviolet.

**13.** Procédé selon la revendication 12, caractérisé en que la polymérisation est effectuée à une température comprise entre 10 ° C et 50 ° C.

**14.** Procédé selon l'une des revendications 1 à 13, caractérisé en ce que le générateur de radicaux libres est un initiateur chimique hydrosoluble.

**15.** Procédé selon la revendication 14, caractérisé en ce que ledit initiateur est choisi parmi les persulfates, les peroxydes, les hydroperoxydes et les composés diazoïques.

**16.** Procédé selon la revendication 15, caractérisé en ce que ledit initiateur est un persulfate de métal alcalin et en ce qu'il utilisé en combinaison avec un réducteur choisi les polyhydrophénols, le sulfite et le bisulfite de sodium, le diméthylaminopropionitrile, les diazomercaptans, les ferricyanures et le sulfate ferreux.

**17.** Procédé selon la revendication 16, caractérisé en ce que ledit réducteur est utilisé à raison de 0,05 à 2% en poids par rapport au monomère hydrosoluble éthyléniquement insaturé.

**18.** Procédé selon l'une des revendications 14 à 17, caractérisé en ce que l'initiateur chimique hydrosoluble est utilisé à raison de 0,05 à 2% en poids par rapport au monomère hydrosoluble éthyléniquement insaturé.

**19.** Procédé selon l'une des revendications 14 à 18, caractérisé en ce que la polymérisation est effectuée à une température comprise entre 20 ° C et la température d'ébullition de l'azéotrope formé entre l'eau et l'hydrocarbure de la phase (2).

**20.** Procédé selon l'une des revendications 14 à 19, caractérisé en ce que la durée de la réaction est comprise entre 10 et 200 minutes.

**21.** Procédé selon l'une des revendications 1 à 20, caractérisé en ce qu'il conduit à un polymère en poudre ayant une capacité d'absorption et de rétention d'eau élevée, à base d'au moins un monomère hydrosoluble éthyléniquement insaturé, la capacité de succion capillaire sous pression après 20 minutes étant au moins égale à 20 ml/g.

**22.** Procédé selon l'une des revendications 1 à 22, caractérisé en ce que le diamètre moyen des particules est compris entre 110 et 220 $\mu$m.

**23.** Procédé selon l'une des revendications 1 à 22, caractérisé en ce qu'un gel obtenu après absorption d'eau saline physiologique par le polymère obtenu possède un module élastique au moins égal à 0,35 N/$cm^2$ (35.000 dynes/$cm^2$).

**24.** Articles capables d'absorber les fluides aqueux, caractérisés en ce qu'ils comprennent au moins un polymère en poudre préparé par le procédé tel que défini à l'une des revendications 1 à 23.

## Claims

### Claims for the following Contracting States : DE, GB, FR, IT, NL, SE, LI, CH

1. Process for preparing a pulverulent polymer having a high capacity for the absorption and retention of water, consisting:
   - in dispersing and suspending an aqueous phase (1) consisting of an aqueous solution of at least one ethylenically unsaturated water-soluble monomer, containing an effective quantity to ensure cross-linking of the target polymer of at least one cross-linking agent which is soluble in the said phase (1) in an oily phase (2) consisting of at least one aliphatic or alicyclic hydrocarbon containing at least 6 carbon atoms and containing an effective quantity of at least one surface-active agent, then
   - in polymerizing the said ethylenically unsaturated water-soluble monomer in the presence of at least one free radical generator,

   characterized in that the surface-active agent is a copolymer containing at least two polymeric components derived from liposoluble complex monocarboxylic acids and another polymeric component residue of a water-soluble compound containing polyoxyalkylene units, the said copolymers being denoted by the general formula:

   $(A\text{-}COO)_m\text{-}B$

   in which:
   - m is an integer equal to at least 2;
   - each polymeric component A has a molecular mass equal to at least 500 and is the residue of a liposoluble complex monocarboxylic acid of general formula :

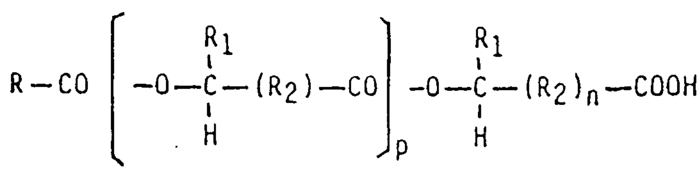

(I)

in which :
- R is hydrogen or else a monovalent or substituted hydrocarbon group,
- $R_1$ is hydrogen or else a monovalent hydrocarbon group containing from 1 to 24 carbon atoms,
- $R_2$ is a $C_1$-$C_{24}$ divalent hydrocarbon group,
- n is equal to 0 or else 1,
- p is an integer ranging from 0 to 200 ; and

each polymeric component B has a molecular mass equal to at least 500 and, in the case where m is equal to 2, is the divalent residue of a water-soluble polyalkylene glycol of general formula :

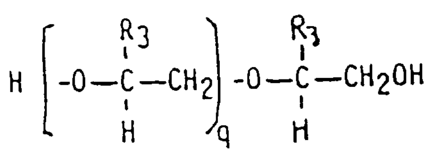

(II)

in which :
- $R_3$ is hydrogen or else an alkyl group containing from 1 to 3 carbon atoms,
- q is an integer ranging from 10 to 500,

or else, in the case where m is greater than 2, is the residue of valency m of a water-soluble polyether polyol of general formula:

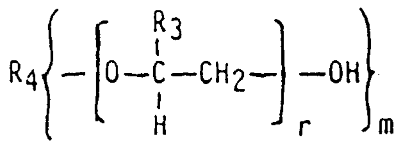

(III)

in which:

- $R_3$ and m have the above meaning,
- r is an integer ranging from 0 to 500, on condition that the total number of units

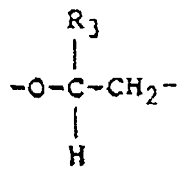

in the molecule is equal to at least 10,
- $R_4$ is the residue of an organic compound containing m hydrogen atoms capable of reacting with an alkylene oxide.

**2.** Process according to Claim 1, characterized in that the weight ratio of phase (2) to phase (1) is lower than or equal to 5.

**3.** Process according to Claim 1, characterized in that the weight ratio of phase (2) to phase (1) is lower than or equal to 0.75.

**4.** Process according to one of Claims 1 to 3, characterized in that the ethylenically unsaturated water-soluble monomer is chosen from acrylic acid and its alkali metal or ammonium salts.

**5.** Process according to Claim 4, characterized in that the ethylenically unsaturated water-soluble monomer is a mixture of a major proportion of alkali metal or ammonium acrylate and a minor proportion of acrylic acid.

**6.** Process according to one of Claims 1 to 5, characterized in that the concentration of the ethylenically unsaturated water-soluble monomer in the aqueous solution of phase (1) is higher than or equal to 40 % by weight.

**7.** Process according to one of Claims 1 to 6, characterized in that the HLB value of the surface-active agent is between 2 and 16.

**8.** Process according to one of Claims 1 to 7, characterized in that the surface-active agent is used in a weight ratio higher than or equal to $2 \times 10^{-4}$ relative to phase (2).

**9.** Process according to one of Claims 1 to 8, characterized in that the surface-active agent is used in a weight ratio not exceeding $2 \times 10^{-2}$ relative to phase (2).

**10.** Process according to one of Claims 1 to 9, characterized in that the crosslinking agent is chosen from compounds containing at least two polymerizable bonds and compounds containing at least one polymerizable double bond and at least one functional group capable of reacting with the ethylenically unsaturated water-soluble monomer.

**11.** Process according to one of Claims 1 to 10, characterized in that the quantity of crosslinking agent is between 0.04 % and 2 % by weight relative to the ethylenically unsaturated water-soluble monomer.

**12.** Process according to one of Claims 1 to 11, characterized in that the free radical generator is chosen from microwaves and beta, gamma and ultraviolet radiations.

**13.** Process according to Claim 12, characterized in that the polymerization is carried out at a temperature of between 10 °C and 50 °C.

**14.** Process according to one of Claims 1 to 13, characterized in that the free radical generator is a water-soluble chemical initiator.

**15.** Process according to Claim 14, characterized in that the said initiator is chosen from persulphates, peroxides, hydroperoxides and diazo compounds.

**16.** Process according to Claim 15, characterized in that the said initiator is an alkali metal persulphate and in that it is employed in combination with a reducing agent chosen from polyhydrophenols, sodium sulphite and bisulphite, dimethylaminopropionitrile, diazomercaptans, ferricyanides and ferrous sulphate.

**17.** Process according to Claim 16, characterized in that the said reducing agent is employed in a proportion of 0.05 to 2 % by weight relative to the ethylenically unsaturated water-soluble monomer.

**18.** Process according to one of Claims 14 to 17, characterized in that the water-soluble chemical initiator is employed in a proportion of 0.05 to 2 % by weight relative to the ethylenically unsaturated water-soluble monomer.

**19.** Process according to one of Claims 14 to 18, characterized in that the polymerization is carried out at a temperature of between 20 °C and the boiling temperature of the azeotrope formed between the water and the hydrocarbon of phase (2).

**20.** Process according to one of Claims 14 to 19, characterized in that the duration of the reaction is between 10 and 200 minutes.

**21.** Pulverulent polymer having a high water absorption and retention capacity, based on at least one ethylenically unsaturated water-soluble monomer, characterized in that its capillary suction capacity under pressure after 20 minutes is equal to at least 20 ml/g, and in that it is capable of being obtained by the process according to one of Claims 1 to 20.

**22.** Pulverulent polymer according to Claim 21, characterized in that the mean particle diameter is between 110 and 220 µm.

**23.** Pulverulent polymer according to either of Claims 21 and 22, characterized in that a gel obtained after absorption of physiological saline water by the said polymer has an elastic modulus equal to at least 0.35 $N/cm^2$ (35,000 $dynes/cm^2$).

**24.** Articles capable of absorbing aqueous fluids, characterized in that they comprise at least one pulverulent polymer according to one of Claims 21 to 23.

**Claims for the following Contracting States : AT, ES**

**1.** Process for preparing a pulverulent polymer having a high capacity for the absorption and retention of water, consisting:

- in dispersing and suspending an aqueous phase (1) consisting of an aqueous solution of at least one ethylenically unsaturated water-soluble monomer, containing an effective quantity to ensure crosslinking of the target polymer of at least one crosslinking agent which is soluble in the said phase (1) in an oily phase (2) consisting of at least one aliphatic or alicyclic hydrocarbon containing at least 6 carbon atoms and containing an effective quantity of at least one surface-active agent, then
- in polymerizing the said ethylenically unsaturated water-soluble monomer in the presence of at least one free radical generator,

characterized in that the surface-active agent is a copolymer containing at least two polymeric components derived from liposoluble complex monocarboxylic acids and another polymeric component residue of a water-soluble compound containing polyoxyalkylene units, the said copolymers being

denoted by the general formula:

(A-COO)$_m$-B

in which:

- m is an integer equal to at least 2;
- each polymeric component A has a molecular mass equal to at least 500 and is the residue of a liposoluble complex monocarboxylic acid of general formula:

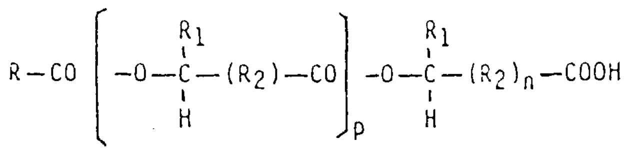

(I)

in which :

- R is hydrogen or else a monovalent or substituted hydrocarbon group,
- $R_1$ is hydrogen or else a monovalent hydrocarbon group containing from 1 to 24 carbon atoms,
- $R_2$ is a $C_1$-$C_{24}$ divalent hydrocarbon group,
- n is equal to 0 or else 1,
- p is an integer ranging from 0 to 200 ; and

each polymeric component B has a molecular mass equal to at least 500 and, in the case where m is equal to 2, is the divalent residue of a water-soluble polyalkylene glycol of general formula :

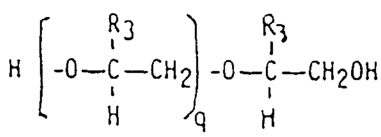

(II)

in which :

- $R_3$ is hydrogen or else an alkyl group containing from 1 to 3 carbon atoms,
- q is an integer ranging from 10 to 500,

or else, in the case where m is greater than 2, is the residue of valency m of a water-soluble polyether polyol of general formula

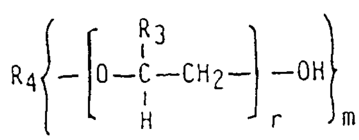

(III)

in which :

- $R_3$ and m have the above meaning,
- r is an integer ranging from 0 to 500, on condition that the total number of units

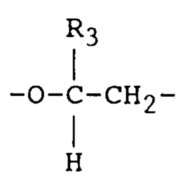

in the molecule is equal to at least 10,

- $R_4$ is the residue of an organic compound containing m hydrogen atoms capable of reacting with an alkylene oxide.

2. Process according to Claim 1, characterized in that the weight ratio of phase (2) to phase (1) is lower than or equal to 5.

3. Process according to Claim 1, characterized in that the weight ratio of phase (2) to phase (1) is lower than or equal to 0.75.

4. Process according to one of Claims 1 to 3, characterized in that the ethylenically unsaturated water-soluble monomer is chosen from acrylic acid and its alkali metal or ammonium salts.

5. Process according to Claim 4, characterized in that the ethylenically unsaturated water-soluble monomer is a mixture of a major proportion of alkali metal or ammonium acrylate and a minor proportion of acrylic acid.

6. Process according to one of Claims 1 to 5, characterized in that the concentration of the ethylenically unsaturated water-soluble monomer in the aqueous solution of phase (1) is higher than or equal to 40 % by weight.

7. Process according to one of Claims 1 to 6, characterized in that the HLB value of the surface-active agent is between 2 and 16.

8. Process according to one of Claims 1 to 7, characterized in that the surface-active agent is used in a weight ratio higher than or equal to $2\times10^{-4}$ relative to phase (2).

9. Process according to one of Claims 1 to 8, characterized in that the surface-active agent is used in a weight ratio not exceeding $2\times10^{-2}$ relative to phase (2).

10. Process according to one of Claims 1 to 9, characterized in that the crosslinking agent is chosen from compounds containing at least two polymerizable bonds and compounds containing at least one polymerizable double bond and at least one functional group capable of reacting with the ethylenically unsaturated water-soluble monomer.

11. Process according to one of Claims 1 to 10, characterized in that the quantity of crosslinking agent is between 0.04 % and 2 % by weight relative to the ethylenically unsaturated water-soluble monomer.

12. Process according to one of Claims 1 to 11, characterized in that the free radical generator is chosen from microwaves and beta, gamma and ultraviolet radiations.

13. Process according to Claim 12, characterized in that the polymerization is carried out at a temperature of between 10 °C and 50 °C.

14. Process according to one of Claims 1 to 13, characterized in that the free radical generator is a water-soluble chemical initiator.

15. Process according to Claim 14, characterized in that the said initiator is chosen from persulphates, peroxides, hydroperoxides and diazo compounds.

16. Process according to Claim 15, characterized in that the said initiator is an alkali metal persulphate and in that it is employed in combination with a reducing agent chosen from polyhydrophenols, sodium sulphite and bisulphite, dimethylaminopropionitrile, diazomercaptans, ferricyanides and ferrous sulphate.

17. Process according to Claim 16, characterized in that the said reducing agent is employed in a proportion of 0.05 to 2 % by weight relative to the ethylenically unsaturated water-soluble monomer.

18. Process according to one of Claims 14 to 17, characterized in that the water-soluble chemical initiator is employed in a proportion of 0.05 to 2 % by weight relative to the ethylenically unsaturated water-soluble monomer.

**19.** Process according to one of Claims 14 to 18, characterized in that the polymerization is carried out at a temperature of between 20 °C and the boiling temperature of the azeotrope formed between the water and the hydrocarbon of phase (2).

**20.** Process according to one of Claims 14 to 19, characterized in that the duration of the reaction is between 10 and 200 minutes.

**21.** Process according to one of claims 1 to 20, characterized in that it produces a pulverulent polymer having a high water absorption and retention capacity, based on at least one ethylenically unsaturated water-soluble monomer, the capillary suction capacity under pressure after 20 minutes being equal to at least 20 ml/g.

**22.** Process according to one of Claims 1 to 21, characterized in that the mean particle diameter is between 110 and 220 µm.

**23.** Process according to one of Claims 1 to 22, characterized in that a gel obtained after absorption of physiological saline water by the polymer obtained has an elastic modulus equal to at least 0.35 $N/cm^2$ (35,000 $dynes/cm^2$).

**24.** Articles capable of absorbing aqueous fluids, characterized in that they comprise at least one pulverulent polymer prepared by the process as defined in one of Claims 1 to 23.

**Patentansprüche**
**Patentansprüche für folgende Vertragsstaaten : DE, GB, FR, IT, NL, SE, LI, CH, BE, LU, GR**

**1.** Verfahren zur Herstellung eines pulverförmigen Polymers mit hoher Absorptionsfähigkeit und hohem Wasserrückhaltevermögen, das darin besteht,

- eine wäßrige Phase (1), die aus einer wäßrigen Lösung von mindestens einem wasserlöslichen, ethylenisch ungesättigten Monomeren besteht, die eine wirksame Menge mindestens eines in dieser Phase (1) löslichen Vernetzungsmittels enthält, um die Vernetzung des gewünschten Polymers zu gewährleisten, mit einer Ölphase (2), die aus mindestens einem aliphatischen oder alicyclischen Kohlenwasserstoff mit wenigstens sechs Kohlenstoffatomen besteht, und die eine wirksame Menge von mindestens einem Tensid enthält, zu dispergieren und in Suspension zu bringen,

und weiter

- das erwähnte wasserlösliche, ethylenisch ungesättigte Monomer in Gegenwart von mindestens einem Bildner freier Radikale zu polymerisieren,

dadurch gekennzeichnet, daß das Tensid ein Copolymer ist, das mindestens zwei Polymerderivate aus komplexen fettlöslichen Monocarbonsäuren und einer anderen Polymerverbindung enthält, die aus einer wasserlöslichen, Polyoxyalkylengruppen enthaltenden Verbindung hervorgegangen ist, wobei die erwähnten Copolymere durch folgende allgemeine Formel dargestellt werden:

$(A - COO)_m - B$

in der

- m eine ganze Zahl von mindestens 2 ist,
- jede Polymerkomponente A ein Molekulargewicht von mindestens 500 besitzt und der Rest einer fettlöslichen komplexen Monocarbonsäure der allgemeinen Formel

$$R-CO\left[-O-\underset{H}{\overset{R_1}{C}}-(R_2)-CO\right]_p-O-\underset{H}{\overset{R_1}{C}}-(R_2)_n-COOH \qquad (I)$$

ist, in der

- R Wasserstoff oder eine einwertige Kohlenwasserstoffgruppe darstellt, die gegebenenfalls substituiert ist,
- $R_1$ Wasserstoff oder eine einwertige Kohlenwasserstoffgruppe darstellt, die 1 bis 24 Kohlenstoffatome enthält,
- $R_2$ eine zweiwertige Kohlenwasserstoffgruppe von $C_1$ bis $C_{24}$ darstellt,
- n gleich 0 oder 1 ist,
- p eine ganze Zahl zwischen 0 und 200 ist,

und

- jede Polymerkomponente B ein Molekulargewicht von mindestens 500 hat und B im Fall m = 2 ein zweiwertiger, wasserlöslicher Polyalkylenglykol-Rest der allgemeinen Formel

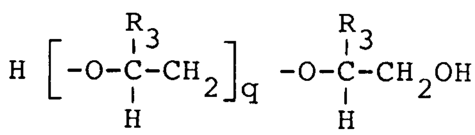

(II)

ist, in der
- $R_3$ Wasserstoff oder eine Alkylgruppe mit 1 bis 3 Kohlenstoffatomen darstellt,
- q eine ganze Zahl zwischen 10 und 500 ist, oder B im Fall m >2 ein m-wertiger, wasserlöslicher Polyetherpolyol-Rest der allgemeinen Formel

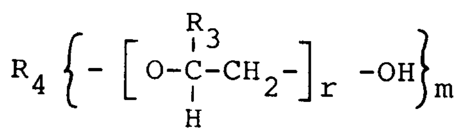

(III)

ist, in der
- $R_3$ und m die oben erwähnte Bedeutung haben,
- r eine ganze Zahl zwischen 0 und 500 ist, unter der Bedingung, daß die Gesamtmenge an Einheiten der Form

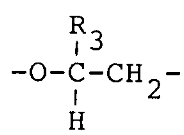

mindestens gleich 10 ist,
- $R_4$ der Rest einer organischen Komponente mit m reaktiven Wasserstoffatomen gegenüber Alkylenoxid ist.

**2.** Verfahren nach Anspruch 1, dadurch gekennzeichnet, daß das Gewichtsverhältnis von Phase (2) zu Phase (1) kleiner oder gleich 5 ist.

**3.** Verfahren nach Anspruch 1, dadurch gekennzeichnet, daß das Gewichtsverhältnis von Phase (2) zu Phase (1) kleiner oder gleich 0,75 ist.

**4.** Verfahren nach einem der Ansprüche 1 bis 3, dadurch gekennzeichnet, daß das wasserlösliche, ethylenisch ungesättigte Monomer unter der Acrylsäure und ihren alkalischen Salzen oder Ammoniumsalzen ausgewählt wird.

**5.** Verfahren nach Anspruch 4, dadurch gekennzeichnet, daß das wasserlösliche, ethylenisch ungesättigte Monomer ein Gemisch mit überproportionalem Anteil von Alkali- oder Ammonium-acrylat und unterproportionalem Anteil von Acrylsäure darstellt.

6. Verfahren nach einem der Ansprüche 1 bis 5, dadurch gekennzeichnet, daß die Konzentration des wasserlöslichen, ethylenisch ungesättigten Monomers in der wäßrigen Lösung der Phase (1) größer oder gleich 40 Gew.% ist.

7. Verfahren nach einem der Ansprüche 1 bis 6, dadurch gekennzeichnet, daß der HLB-Wert des Tensids zwischen 2 und 16 liegt.

8. Verfahren nach einem der Ansprüche 1 bis 7, dadurch gekennzeichnet, daß das Tensid in einem Gewichtsverhältnis größer oder gleich 2 x $10^{-4}$, bezogen auf Phase (2) eingesetzt wird.

9. Verfahren nach einem der Ansprüche 1 bis 8, dadurch gekennzeichnet, daß das Tensid in einem Gewichtsverhältnis nicht über 2 x $10^{-2}$, bezogen auf Phase (2) eingesetzt wird.

10. Verfahren nach einem der Ansprüche 1 bis 9, dadurch gekennzeichnet, daß das Vernetzungsmittel aus Verbindungen mit wenigstens zwei polymerisierbaren Bindungen und aus Verbindungen mit mindestens einer polymerisierbaren Doppelbindung und mindestens einer funktionellen Gruppe, die mit dem wasserlöslichen, ethylenisch ungesättigten Monomer eine Reaktion eingeht, gewählt wird.

11. Verfahren nach einem der Ansprüche 1 bis 10, dadurch gekennzeichnet, daß die Menge des Vernetzungsmittels im Verhältnis zum wasserlöslichen, ethylenisch ungesättigten Monomer zwischen 0,04 und 2 Gew.% liegt.

12. Verfahren nach einem der Ansprüche 1 bis 11, dadurch gekennzeichnet, daß als Bildner für freie Radikale Mikrowellen, Beta-Strahlung, Gamma-Strahlung und ultraviolette Strahlung gewählt wird.

13. Verfahren nach Anspruch 12, dadurch gekennzeichnet, daß die Polymerisation bei einer Temperatur zwischen 10 und 50 °C erfolgt.

14. Verfahren nach einem der Ansprüche 1 bis 13, dadurch gekennzeichnet, daß der Bildner für freie Radikale ein chemischer, wasserlöslicher Initiator ist.

15. Verfahren nach Anspruch 14, dadurch gekennzeichnet, daß der Initiator aus den Persulfaten, den Peroxiden, den Hydroperoxiden und den Diazoverbindungen gewählt wird.

16. Verfahren nach Anspruch 15, dadurch gekennzeichnet, daß der Initiator ein Alkalimetallpersulfat ist, und daß er zusammen mit einem Reduktionsmittel eingesetzt wird, das aus den Polyhydrophenolen, dem Natriumsulfit und dem Natriumbisulfit, dem Dimethylaminopropionitril, den Diazomercaptanen, den Eisen(II)-cyaniden und dem Eisen(II)-sulfat gewählt wird.

17. Verfahren nach Anspruch 16, dadurch gekennzeichnet, daß von dem Reduktionsmittel 0,05 bis 2 Gew.%, bezogen auf das wasserlösliche, ethylenisch ungesättigte Monomer eingesetzt werden.

18. Verfahren nach einem der Ansprüche 14 bis 17, dadurch gekennzeichnet, daß der chemische, wasserlösliche Initiator in einer Menge von 0,05 bis 2 Gew.%, bezogen auf das wasserlösliche, ethylenisch ungesättigte Monomer eingesetzt wird.

19. Verfahren nach einem der Ansprüche 14 bis 18, dadurch gekennzeichnet, daß die Polymerisation bei einer Temperatur zwischen 20 °C und der Siedetemperatur des Azeotrops, welches sich zwischen dem Wasser und dem Kohlenwasserstoff der Phase (2) gebildet hat, erfolgt.

20. Verfahren nach einem der Ansprüche 14 bis 19, dadurch gekennzeichnet, daß die Reaktionsdauer 10 bis 200 Minuten beträgt.

21. Pulverförmiges Polymer mit hoher Absorptionsfähigkeit und hohem Wasserrückhaltevermögen, das mindestens ein wasserlösliches, ethylenisch ungesättigtes Monomer enthält, dadurch gekennzeichnet, daß die kapillare Saugfähigkeit unter Druck nach 20 Minuten mindestens 20 ml/g beträgt, und daß es durch das Verfahren nach einem der Ansprüche 1 bis 20 gewonnen werden kann.

**22.** Pulverförmiges Polymer nach Anspruch 21, dadurch gekennzeichnet, daß der mittlere Durchmesser der Teilchen zwischen 110 und 220 µm liegt.

**23.** Pulverförmiges Polymer nach einem der Ansprüche 21 bis 22, dadurch gekennzeichnet, daß ein Gel, das nach Absorption von physiologischer Kochsalzlösung durch das erwähnte Polymer erhalten wurde, ein Elastizitätsmodul von mindestens 0,35 $N/cm^2$ (35.000 $dyn/cm^2$) besitzt.

**24.** Produkte mit der Fähigkeit, wäßrige Flüssigkeiten zu absorbieren, dadurch gekennzeichnet, daß sie mindestens ein pulverförmiges Polymer nach einem der Ansprüche 21 bis 23 enthalten.

**Patentansprüche für folgende Vertragsstaaten : AT, ES**

**1.** Verfahren zur Herstellung eines pulverförmigen Polymers mit hoher Absorptionsfähigkeit und hohem Wasserrückhaltevermögen, das darin besteht,
- eine wäßrige Phase (1), die aus einer wäßrigen Lösung von mindestens einem wasserlöslichen, ethylenisch ungesättigten Monomeren besteht, die eine wirksame Menge mindestens eines in dieser Phase (1) löslichen Vernetzungsmittels enthält, um die Vernetzung des gewünschten Polymers zu gewährleisten, mit einer Ölphase (2), die aus mindestens einem aliphatischen oder alicyclischen Kohlenwasserstoff mit wenigstens sechs Kohlenstoffatomen besteht, und die eine wirksame Menge von mindestens einem Tensid enthält, zu dispergieren und in Suspension zu bringen,

und weiter
- das erwähnte wasserlösliche, ethylenisch ungesättigte Monomer in Gegenwart von mindestens einem Bildner freier Radikale zu polymerisieren,

dadurch gekennzeichnet, daß das Tensid ein Copolymer ist, das mindestens zwei Polymerderivate aus komplexen fettlöslichen Monocarbonsäuren und einer anderen Polymerverbindung enthält, die aus einer wasserlöslichen, Polyoxyalkylengruppen enthaltenden Verbindung hervorgegangen ist, wobei die erwähnten Copolymere durch folgende allgemeine Formel dargestellt werden:

$(A - COO)_m -B$

in der
- m eine ganze Zahl von mindestens 2 ist,
- jede Polymerkomponente A ein Molekulargewicht von mindestens 500 besitzt und der Rest einer fettlöslichen komplexen Monocarbonsäure der allgemeinen Formel

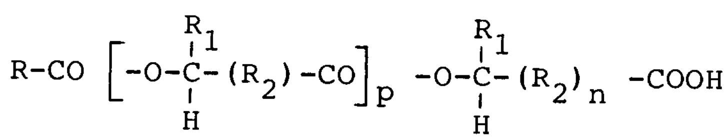 (I)

ist, in der
- R Wasserstoff oder eine einwertige Kohlenwasserstoffgruppe darstellt, die gegebenenfalls substituiert ist,
- $R_1$ Wasserstoff oder eine einwertige Kohlenwasserstoffgruppe darstellt, die 1 bis 24 Kohlenstoffatome enthält,
- $R_2$ eine zweiwertige Kohlenwasserstoffgruppe von $C_1$ bis $C_{24}$ darstellt,
- n gleich 0 oder 1 ist,
- p eine ganze Zahl zwischen 0 und 200 ist,

und
- jede Polymerkomponente B ein Molekulargewicht von mindestens 500 hat und B im Fall m = 2 ein zweiwertiger, wasserlöslicher Polyalkylenglykol-Rest der allgemeinen Formel

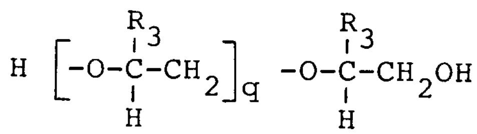 (II)

ist, in der

- $R_3$ Wasserstoff oder eine Alkylgruppe mit 1 bis 3 Kohlenstoffatomen darstellt,
- q eine ganze Zahl zwischen 10 und 500 ist, oder B im Fall m>2 ein m-wertiger, wasserlöslicher Polyetherpolyol-Rest der allgemeinen Formel

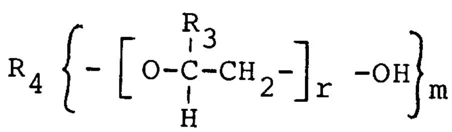 (III)

ist, in der

- $R_3$ und m die oben erwähnte Bedeutung haben,
- r eine ganze Zahl zwischen 0 und 500 ist, unter der Bedingung, daß die Gesamtmenge an Einheiten der Form

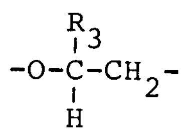

mindestens gleich 10 ist,

- $R_4$ der Rest einer organischen Komponente mit m reaktiven Wasserstoffatomen gegenüber Alkylenoxid ist.

**2.** Verfahren nach Anspruch 1, dadurch gekennzeichnet, daß das Gewichtsverhältnis von Phase (2) zu Phase (1) kleiner oder gleich 5 ist.

**3.** Verfahren nach Anspruch 1, dadurch gekennzeichnet, daß das Gewichtsverhältnis von Phase (2) zu Phase (1) kleiner oder gleich 0,75 ist.

**4.** Verfahren nach einem der Ansprüche 1 bis 3, dadurch gekennzeichnet, daß das wasserlösliche, ethylenisch ungesättigte Monomer unter der Acrylsäure und ihren alkalischen Salzen oder Ammoniumsalzen ausgewählt wird.

**5.** Verfahren nach Anspruch 4, dadurch gekennzeichnet, daß das wasserlösliche, ethylenisch ungesättigte Monomer ein Gemisch mit überproportionalem Anteil von Alkali- oder Ammonium-acrylat und unterproportionalem Anteil von Acrylsäure darstellt.

**6.** Verfahren nach einem der Ansprüche 1 bis 5, dadurch gekennzeichnet, daß die Konzentration des wasserlöslichen, ethylenisch ungesättigten Monomers in der wäßrigen Lösung der Phase (1) größer oder gleich 40 Gew.% ist.

**7.** Verfahren nach einem der Ansprüche 1 bis 6, dadurch gekennzeichnet, daß der HLB-Wert des Tensids zwischen 2 und 16 liegt.

8. Verfahren nach einem der Ansprüche 1 bis 7, dadurch gekennzeichnet, daß das Tensid in einem Gewichtsverhältnis größer oder gleich 2 x $10^{-4}$, bezogen auf Phase (2) eingesetzt wird.

9. Verfahren nach einem der Ansprüche 1 bis 8, dadurch gekennzeichnet, daß das Tensid in einem Gewichtsverhältnis nicht über 2 x $10^{-2}$, bezogen auf Phase (2) eingesetzt wird.

10. Verfahren nach einem der Ansprüche 1 bis 9, dadurch gekennzeichnet, daß das Vernetzungsmittel aus Verbindungen mit wenigstens zwei polymerisierbaren Bindungen und aus Verbindungen mit mindestens einer polymerisierbaren Doppelbindung und mindestens einer funktionellen Gruppe, die mit dem wasserlöslichen, ethylenisch ungesättigten Monomer eine Reaktion eingeht, gewählt wird.

11. Verfahren nach einem der Ansprüche 1 bis 10, dadurch gekennzeichnet, daß die Menge des Vernetzungsmittels im Verhältnis zum wasserlöslichen, ethylenisch ungesättigten Monomer zwischen 0,04 und 2 Gew.% liegt.

12. Verfahren nach einem der Ansprüche 1 bis 11, dadurch gekennzeichnet, daß als Bildner für freie Radikale Mikrowellen, Beta-Strahlung, Gamma-Strahlung und ultraviolette Strahlung gewählt wird.

13. Verfahren nach Anspruch 12, dadurch gekennzeichnet, daß die Polymerisation bei einer Temperatur zwischen 10 und 50 °C erfolgt.

14. Verfahren nach einem der Ansprüche 1 bis 13, dadurch gekennzeichnet, daß der Bildner für freie Radikale ein chemischer, wasserlöslicher Initiator ist.

15. Verfahren nach Anspruch 14, dadurch gekennzeichnet, daß der Initiator aus den Persulfaten, den Peroxiden, den Hydroperoxiden und den Diazoverbindungen gewählt wird.

16. Verfahren nach Anspruch 15, dadurch gekennzeichnet, daß der Initiator ein Alkalimetallpersulfat ist, und daß er zusammen mit einem Reduktionsmittel eingesetzt wird, das aus den Polyhydrophenolen, dem Natriumsulfit und dem Natriumbisulfit, dem Dimethylaminopropionitril, den Diazomercaptanen, den Eisen(II)-cyaniden und dem Eisen(II)-sulfat gewählt wird.

17. Verfahren nach Anspruch 16, dadurch gekennzeichnet, daß von dem Reduktionsmittel 0,05 bis 2 Gew.%, bezogen auf das wasserlösliche, ethylenisch ungesättigte Monomer eingesetzt werden.

18. Verfahren nach einem der Ansprüche 14 bis 17, dadurch gekennzeichnet, daß der chemische, wasserlösliche Initiator in einer Menge von 0,05 bis 2 Gew.%, bezogen auf das wasserlösliche, ethylenisch ungesättigte Monomer eingesetzt wird.

19. Verfahren nach einem der Ansprüche 14 bis 18, dadurch gekennzeichnet, daß die Polymerisation bei einer Temperatur zwischen 20 °C und der Siedetemperatur des Azeotrops, welches sich zwischen dem Wasser und dem Kohlenwasserstoff der Phase (2) gebildet hat, erfolgt.

20. Verfahren nach einem der Ansprüche 14 bis 19, dadurch gekennzeichnet, daß die Reaktionsdauer 10 bis 200 Minuten beträgt.

21. Verfahren nach einem der Ansprüche 1 bis 20, dadurch gekennzeichnet, daß es zu einem pulverförmigem Polymer mit hoher Absorptionsfähigkeit und hohem Wasserrückhaltevermögen führt, das mindestens ein wasserlösliches, ethylenisch ungesättigtes Monomer enthält, wobei die kapillare Saugfähigkeit unter Druck nach 20 Minuten mindestens 20 ml/g beträgt.

22. Verfahren nach einem der Ansprüche 1 bis 21, dadurch gekennzeichnet, daß der mittlere Durchmesser der Teilchen zwischen 110 und 220 $\mu$m liegt.

23. Verfahren nach einem der Ansprüche 1 bis 22, dadurch gekennzeichnet, daß ein Gel, das nach Absorption von physiologischer Kochsalzlösung durch das erwähnte Polymer erhalten wurde, ein Elastizitätsmodul von mindestens 0,35 N/$cm^2$ (35.000 dyn/$cm^2$) besitzt.

**24.** Produkte mit der Fähigkeit, wäßrige Flüssigkeiten zu absorbieren, dadurch gekennzeichnet, daß sie mindestens ein pulverförmiges Polymer nach einem der Ansprüche 21 bis 23 enthalten.

FIGURE UNIQUE